# FASCICULE DE BREVET EUROPEEN

(11) **EP 2 625 161 B1**
(45) Date de publication et mention de la délivrance du brevet: **28.05.2014**
(21) Numéro de dépôt: 11779783.7
(22) Date de dépôt: 04.10.2011
(51) Int. Cl.: C07C 227/18, C07C 271/02, C07C 321/14

(54) **PROCÈDE DE PRÉPARATION D'UN ACIDE AMINE A PARTIR DE 2-AMINOBUTYROLACTONE**
VERFAHREN FÜR DIE ZUBEREITUNG EINER AMINOSÄURE AUS 2-AMINOBUTYROLACTON
METHOD FOR PREPARING AN AMINO ACID FROM 2-AMINOBUTYROLACTONE

(30) Priorité: 05.10.2010 FR 1058069
(43) Date de publication de la demande: 14.08.2013
(73) Titulaire: Adisseo France S.A.S., 92160 Antony (FR)
(72) Inventeur: HUET, Robert, F-75015 Paris (FR); JOERGER, Jean-Michel, F-69100 Villeurbanne (FR); HENRYON, Vivien, F-69007 Lyon (FR)
(74) Mandataire: Delorme, Nicolas
(86) Numéro de dépôt international: PCT/FR2011/052302
(87) Numéro de publication internationale: WO 2012/045967

(56) Documents cités:
- GB-A- 651 165
- US-A1- 2008 146 840

## Description

La présente invention concerne un procédé de préparation d'un acide aminé à partir de la 2-aminobutyrolactone (2ABL).

Il existe une conversion classique de la 2ABL en méthionine par réaction du méthanethiolate de sodium (CH₃SNa), telle que décrite dans Chem. Ber. (1950) 83, 265. Cette synthèse est réalisée en une seule étape dans du toluène, à 160°C, sur une durée d'une heure. Son rendement n'est cependant que de 44%.

Selon US2008/0146840A1, on connaît un procédé d'obtention de la méthionine à partir d'homosérine, comprenant les étapes consistant à :
a) effectuer une réaction de N-acylation et cyclisation concomitantes de l'homosérine,
b) à faire réagir le méthylmercaptan avec le N-acétamide de 2-aminobutyrolactone obtenu à l'étape a), en présence d'un catalyseur acide ou basique, pour obtenir le N-acétamide de méthionine, et
c) à hydrolyser le N-acétamide de méthionine en méthionine.

Ce procédé conduit à des rendements plus élevés, mais il présente l'inconvénient de nécessiter trois étapes, devant chacune recourir à la purification des intermédiaires formés.

Par ailleurs, l'homosérine est un composé très disponible. Elle peut être obtenue par voie chimique. Elle peut aussi être obtenue par fermentation microbiologique de sucres ; une large bibliographie existe sur ce sujet. Cette source est d'autant plus attrayante qu'on obtient l'isomère L de l'homosérine. Comme le montre l'art antérieur, l'étape de cyclisation de l'homosérine en 2ABL, protégée ou non, est efficace, mais on se heurte à des rendements beaucoup trop faibles de l'étape ultime de conversion en méthionine ou à un process trop lourd, pour en envisager une synthèse à grande échelle.

GB 651 165 A décrit (example 1) un procédé de préparation de méthionine par réaction de la 2-aminobutyro-lactone (2ABL) avec MeSNa à 180°C, suivi par l'addition d'acide acétique.

Les auteurs de la présente invention ont découvert un groupement protecteur de la fonction amine de la 2ABL, ainsi que le réactif en permettant le placement, ledit groupement étant introduit et retiré facilement, et ce de manière réversible. Ils ont de ce fait révélé une application majeure de l'homosérine dans la préparation d'acides aminés.

L'invention apporte un procédé de préparation d'un acide aminé, ou de ses sels, à partir de la 2-aminobutyrolactone (2ABL), ledit acide aminé répondant à la formule 1, XCH₂CH₂CHNH₂COOH, où X est tel que X-représente un ion nucléophile, qui comprend les étapes suivantes :
- On réalise la N-carboxylation de la 2-aminobutyrolactone (2ABL) par du dioxyde de carbone, et
- On fait réagir le N-carboxyl de la 2ABL ainsi obtenu avec un réactif XH ou ses sels, et on acidifie.

Comme l'illustreront les exemples, les rendements de déprotection rendent possible une synthèse industrielle de la méthionine à partir de 2ABL. La synthèse industrielle de nombreux autres acides aminés, tels que ceux répondant à la formule I ci-dessus est tout aussi préconisable. Par acide aminé, on entend tout acide aminé comportant un carbone asymétrique porteur d'un groupe -NH₂, d'un groupe -COOH, de -H et d'une chaîne latérale de forme -CH₂CH₂X, X étant apporté selon l'invention par le réactif XH ou son sel, ou de forme -CH₂CH₂Y, où Y représente un groupe résultant d'une conversion de X par exemple par hydrolyse. A titre d'exemples préférés, on peut citer en plus de la méthionine, la sélénométhionine, l'homocystéine et la glutamine. Ce procédé permet aussi de préparer l'homocystéine, celle-ci étant obtenue avec son dimère.

Le carbone porteur du groupement aminé et du groupement carboxylique étant asymétrique, on entend par acide aminé, chacun de ses isomères, L- ou D-, ou leurs mélanges et notamment le racémique. On obtient l'isomère ou le mélange d'isomères recherché de l'acide aminé en partant de l'isomère ou du mélange d'isomères correspondant de la 2ABL, et en amont de l'homosérine, le procédé objet de l'invention n'affectant pas la configuration des entités.

Un autre objet de l'invention est t le N-carboxyl-2-aminobutyrolactone. Il s'agit du composé intermédiaire obtenu à l'issue de l'étape de N-carboxylation de la 2ABL. Bien entendu, son application ne se limite pas à cette synthèse.

La réaction de N-carboxylation est avantageusement et simplement réalisée en présence d'un bullage de CO₂ gazeux. De préférence, elle est conduite dans un solvant polaire aprotique. A titre d'exemple, il est choisi parmi le diméthylsulfoxyde et la N-méthylpyrrolidone (NMP). Elle peut aussi être conduite avec du CO₂ super critique.

Cette étape est de préférence menée à une température comprise entre 0 et 50°C.

La seconde étape du procédé met en jeu un réactif XH ou un sel de celui-ci. X est défini tel que X- est un ion, constitué de un ou de plusieurs atomes, qui est nucléophile. Plus spécifiquement, il est capable d'agir sur le carbone en beta de la fonction amine protégée, afin d'ouvrir le cycle lactone. Il comprend un atome riche en électrons généralement choisi parmi les atomes de soufre, de sélénium, d'oxygène, de carbone, ledit atome étant lié à au moins l'un de l'hydrogène, un alkyle en C1-C6, linéaire ou ramifié, l'azote. A titre d'exemples, X- est choisi parmi CH₃S- et CH₃Se⁻ pour obtenir la méthionine et la sélénométhionine, respectivement, parmi SH- et SeH- pour obtenir l'homocystéine et l'acide 2-amino-4-SeH-thiobutyrique, respectivement. Il peut aussi être CN- pour l'obtention de l'acide 2-amino-4-cyano-thiobutyrique ; celui-ci sera avantageusement hydrolysé en groupe amide pour conduire à la glutamine.

Les sels de XH sont avantageusement choisis parmi les sels métalliques, par exemple sels de métaux alcalins et alcalino-terreux.

La concentration du réactif XH ou son sel est avantageusement en excès par rapport à la 2-ABL, elle représente de préférence de 3 à 30% en pourcentage poids/poids de la masse totale du milieu réactionnel. Cette étape se déroule de préférence à une température variant de 100 à 200°C. Avantageusement, la température est de l'ordre de 130°C.

Le procédé de l'invention peut être mis en oeuvre sur une période variant de 5 minutes à 3 heures.

Comme indiqué précédemment, ce procédé permet d'obtenir un acide aminé ou ses sels. Ces sels sont avantageusement choisis parmi les sels de sodium, de lithium, de calcium, de zinc. Pour obtenir un sel, on choisira le sel approprié du réactif XH.

L'invention et ses avantages sont ci-après illustrés à l'appui des exemples suivants décrivant la préparation de méthionine selon les exemples 1 et 2, de l'homocystéine et son dimère selon l'exemple 3, et de sélénométhionine selon les exemples 4, 5, 6 et 7, à partir de 2ABL.

Les exemples 1 et 2 décrivent la préparation de méthionine directement à partir de 2ABL (exemple 1) et à partir de 2ABL en passant par l'intermédiaire 2ABL-N-carboxylée (ou carbonatée), c'est-à-dire selon l'invention (exemple 2), d'après le schéma suivant :

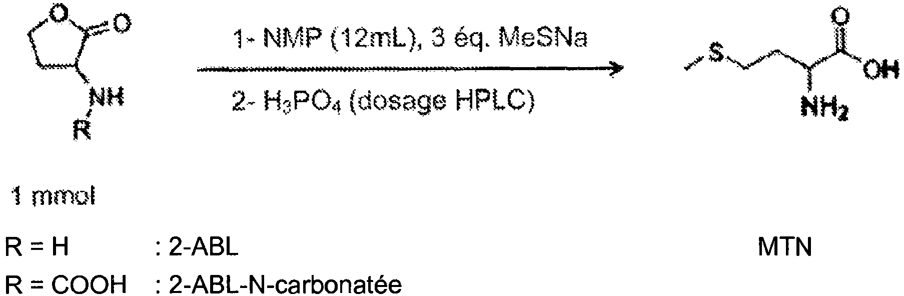

### Exemple 1 : préparation de méthionine directement à partir de 2ABL

Cet essai est réalisé à l'échelle d'1 mmole de 2-ABL. La 2-ABL est placée en solution dans 1,2 mL de NMP. L'agitation est maintenue à 20°C pendant 10 min, puis 10,8 mL de NMP et 3 éq. de MeSNa sont ajoutés. Le milieu réactionnel placé sous agitation est chauffé à 150°C pendant 1 heure. Le milieu est hydrolysé par simple dilution dans le solvant HPLC.

### Exemple 2 : préparation de méthionine selon l'invention

Le schéma réactionnel est le suivant :

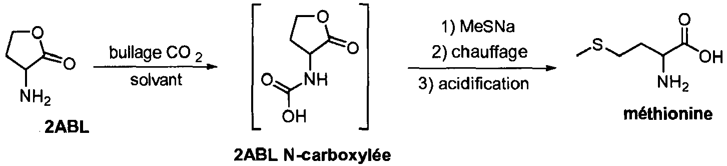

Les conditions opératoires de l'exemple 1 sont reproduites à l'identique, à la seule différence près qu'un bullage de CO₂ est réalisé à 20°C pendant 10 min.

Les résultats obtenus sont présentés dans le tableau 1 suivant :

| Exemple | Substrat | Rendement en % (HPLC) | | |
|---|---|---|---|---|
| | | Méthionine | Dicétopipérazine | Homosérine |
| 1 | 2ABL | 17 | 19 | 29 |
| 2 | 2ABL-N-carboxylée | 80 | 16 | <1 |

On observe un quadruplement du rendement en méthionine préparée par le procédé de l'invention.

L'exemple 3 décrit la préparation de l'homocystéine à partir de 2ABL selon l'invention

### Exemple 3 : Préparation de l'homocystéine selon l'invention

Le schéma réactionnel est le suivant :

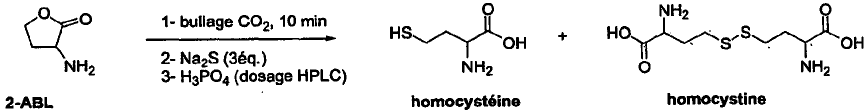

Le procédé de l'invention permet aussi d'obtenir le dimère de l'homocystéine (nommé l'homocystine).

Cet exemple est réalisé à l'échelle d'1 mmol de 2ABL dans un pilulier avec une agitation magnétique. La 2-ABL est placée en solution dans 1,2 mL de NMP. Le bullage du CO₂ est effectué à 20°C pendant 10 min, puis 10,8 mL de NMP et 3 équivalents de Na₂S sont ajoutés. Le milieu réactionnel placé sous agitation est chauffé progressivement jusqu'à 90°C. Après 30 min, le milieu est hydrolysé par simple dilution dans le solvant HPLC.

Ces conditions permettent de former de l'homocystéine et son dimère. Les meilleures performances sont obtenues à une température de 90°C. Après un temps de réaction de 30 min, la réaction est terminée. A une température supérieure et en présence de 3 équivalents de Na₂S, l'homocystine est favorisée. L'excès de Na₂S accélère la dimérisation de l'homocystéine

Les rendements (HPLC) suivants sont obtenus :

| | |
|---|---|
| Homocystéine | 36% |
| Homocystine | 14% |
| Dicétopipérazine | 2% |

Les exemples 4, 5, 6 et 7 décrivent la préparation de sélénométhionine directement à partir de 2ABL (exemples 4 et 6) et à partir de 2ABL en passant par l'intermédiaire 2ABL-N-carboxylée, c'est-à-dire selon l'invention (exemples 5 et 7), par réaction avec MeSeNa ou MeSeLi.

Selon les exemples 4 et 5, le schéma est le suivant :

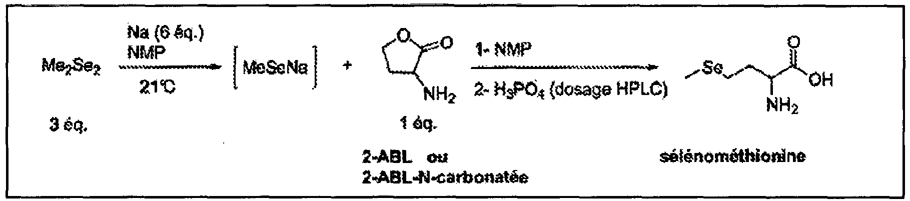

### Exemple 4 : préparation de sélénométhionine directement à partir de 2ABL

Cet exemple est réalisé à l'échelle de 50 mg de Na, dans un pilulier de 4 mL avec une agitation magnétique à 20°C. Le Me₂Se₂ est placé en solution dans 2 mL de NMP puis à 20°C, 50 mg de Na sont additionnés. La 2-ABL est placée en solution dans 870 µL de NMP. L'agitation est maintenue à 20°C pendant 10 min, puis 10,8 mL de NMP et la solution de MeSeNa dans la NMP sont ajoutés. Le milieu réactionnel placé sous agitation est chauffé à 90°C pendant 1 heure. Le milieu réactionnel est hydrolysé par simple dilution dans le solvant HPLC.

### Exemple 5 : préparation de sélénométhionine selon l'invention

Les conditions opératoires de l'exemple 4 sont reproduites à l'identique, à la seule différence près qu'un bullage de CO₂ est réalisé.

Les résultats obtenus sont présentés dans le tableau 2 suivant :

| Exemple | Substrat | Rendement en % (HPLC) | |
|---|---|---|---|
| | | Sélénométhionine | Dicétopipérazine |
| 4 | 2ABL | 17 | 2 |
| 5 | 2ABL-N-carboxylée | 51 | 2 |

Selon les exemples 6 et 7, le schéma est le suivant :

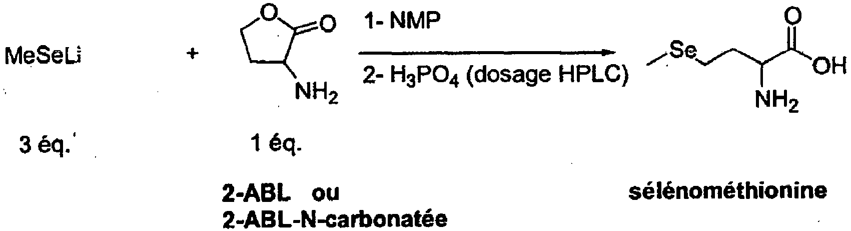

MeSeLi est préparé selon la synthèse décrite dans M. Tiecco et al., Synthetic Communications, 1983, 13, 617.

### Exemple 6 : préparation de méthionine directement à partir de 2ABL

Cet exemple est réalisé à l'échelle d'1 mmol de 2ABL dans un pilulier avec une agitation magnétique à 20°C. La 2-ABL est placée en solution dans 1.2 mL de NMP. L'agitation est maintenue à 20°C pendant 10 min, puis 10,8 mL de NMP et 2,43 mL de MeSeLi en solution dans le THF sont ajoutés. Le milieu réactionnel placé sous agitation est chauffé pendant 1 heure à 60°C. Le milieu réactionnel est hydrolysé par simple dilution dans le solvant HPLC.

### Exemple 7 ; préparation de sélénométhionine selon l'invention

Les conditions opératoires de l'exemple 6 sont reproduites à l'identique, aux seules différences près qu'un bullage de CO₂ est réalisé à 20°C pendant 10 min et que la température de réaction est portée à 90°C.

Les résultats obtenus sont présentés dans le tableau 3 suivant :

| Exemple | Substrat | Température (°C) | Rendement en % (HPLC) | |
|---|---|---|---|---|
| | | | Sélénométhionine | Dicétopipérazine |
| 6 | 2ABL | 60 | 60 | 5 |
| 7 | 2ABL-N-carboxylée | 90 | 92 | 5 |

Il ressort de l'ensemble de ces exemples que le procédé constitue une voie très performante d'obtention d'acides aminés à partir de la 2ABL et en cela une nouvelle exploitation intéressante de l'homosérine.

Le composé intermédiaire N-carboxyl-2-aminobutyrolactone, un des objets de l'invention, formé notamment aux exemples 2, 3, 5 et 7 a été analysé. Ces analyses sont les suivantes :
Spectre RMN du proton (fréquence : 250MHz, solvant dmso-d6) : 2.16 ppm (multiplet, 1 H), 2.28-2.44 (multiplet, 1 H), 4.08-4.22 (multiplet, 1 H), 4.23-4.38 (multiplet, 2H), 7.25 (doublet, J = 8.2 Hz, 1 H)

## Revendications

1. Procédé de préparation d'un acide aminé, ou de ses sels, à partir de la 2-aminobutyrolactone (2ABL), ledit acide aminé répondant à la formule I, XCH₂CH₂CHNH₂COOH, où X est tel que X- représente un ion nucléophile, **caractérisé en ce qu'**il comprend les étapes suivantes :
On réalise la N-carboxylation de la 2-aminobutyrolactone (2ABL) par du dioxyde de carbone, et
On fait réagir le N-carboxyl de la 2ABL ainsi obtenu avec un réactif XH ou ses sels et on acidifie.

2. Procédé selon la revendication 1, **caractérisé en ce que** X- est choisi parmi CH₃S-, CH₃Se-, SH-, SeH-, CN⁻.

3. Procédé selon la revendication 1 ou 2, **caractérisé en ce qu'**on obtient un acide aminé choisi parmi la méthionine et la sélénométhionine.

4. Procédé selon la revendication 1 ou 2, **caractérisé en ce qu'**on obtient l'homocystéine et son dimère.

5. Procédé selon l'une quelconque des revendications 1 à 4, **caractérisé en ce que** la N-carboxylation est réalisée dans un solvant polaire aprotique.

6. Procédé selon la revendication 5, **caractérisé en ce que** le solvant est choisi parmi le diméthylsulfoxyde et la N-méthylpyrrolidone.

7. Procédé selon l'une quelconque des revendications 1 à 6, **caractérisé en ce que** l'on prépare l'isomère L de l'acide aminé, l'isomère D ou les mélanges de ceux-ci et notamment le racémique, à partir de la forme correspondante de la 2ABL.

8. Procédé selon l'une quelconque des revendications 1 à 7, **caractérisé en ce que** la 2ABL est obtenue à partir de l'homosérine.

9. Procédé selon la revendication 8, **caractérisé en ce que** l'homosérine est l'isomère L et est obtenue par fermentation microbiologique de sucres d'origine naturelle.

10. Procédé selon l'une quelconque des revendications 1 à 9, **caractérisé en ce que** les sels de l'acide aminé sont choisis parmi les sels de sodium, de lithium, de calcium, de zinc.

11. N-Carboxyl-2-aminobutyrolactone et ses sels.

## Patentansprüche

1. Verfahren zur Herstellung einer Aminosäure oder ihrer Salze, ausgehend von 2-Aminobutyrolacton (2ABL), wobei die Aminosäure der Formel I, XCH₂CH₂CHNH₂COOH, entspricht, wobei X derart ist, dass X- ein nucleophiles Ion darstellt, **dadurch gekennzeichnet, dass** es die folgenden Schritte umfasst:
Durchführen der N-Carboxylierung des 2-Aminobutyrolactons (2ABL) durch Kohlendioxyd, und
Reagieren des N-Carboxyls des so erhaltenen 2ABL mit einem XH-Reagens oder seinen Salzen und Versäuern.

2. Verfahren nach Anspruch 1, **dadurch gekennzeichnet, dass** X-ausgewählt ist aus CH₃S-, CH₃Se-, SH-, SeH⁻, CN⁻.

3. Verfahren nach Anspruch 1 oder 2, **dadurch gekennzeichnet, dass** eine Aminosäure erhalten wird, ausgewählt aus Methionin und Selenomehtionin.

4. Verfahren nach Anspruch 1 oder 2, **dadurch gekennzeichnet, dass** Homocystein und sein Dimer erhalten wird.

5. Verfahren nach einem der Ansprüche 1 bis 4, **dadurch gekennzeichnet, dass** die N-Carboxylierung in einem aprotisch-polaren Lösemittel durchgeführt wird.

6. Verfahren nach Anspruch 5, **dadurch gekennzeichnet, dass** das Lösemittel ausgewählt ist aus Dimethylsulfoxyd und N-Methylpyrrolidon.

7. Verfahren nach einem der Ansprüche 1 bis 6, **dadurch gekennzeichnet, dass** das L-Isomer der Aminosäure, das D-Isomer oder die Mischungen dieser und insbesondere die racemische, ausgehend von der entsprechenden Form des 2ABL zubereitet wird.

8. Verfahren nach einem der Ansprüche 1 bis 7, **dadurch gekennzeichnet, dass** das 2ABL ausgehend von Homoserin erhalten wird.

9. Verfahren nach Anspruch 8, **dadurch gekennzeichnet, dass** das Homoserin das L-Isomer ist und durch mikrobiologische Fermentierung von Zuckern natürlichen Ursprungs erhalten wird.

10. Verfahren nach einem der Ansprüche 1 bis 9, **dadurch gekennzeichnet, dass** die Salze der Aminosäure ausgewählt sind aus dem Natriumsalz, dem Litihumsalz, dem Calciumsalz, dem Zinksalz.

11. N-Carboxyl-2-aminobutyrolacton und seine Salze.

## Claims

1. A method for preparing an amino acid, or the salts thereof, from 2-aminobutyrolactone (2ABL), said amino acid whose chemical formula is I, XCH₂CH₂CHNH₂COOH, where X is such that X- represents a nucleophilic ion, **characterized in that** it comprises the following steps:
It is achieved the N-carboxylation of the 2-aminobutyrolactone (2ABL) by carbon dioxide, and
It is made to react N-carboxyl of the thus obtained 2ABL with a reagent XH or the salts thereof and it is acidified.

2. The method according to claim 1, **characterized in that** X- is selected from among CH₃S⁻,CH₃Se⁻, SH⁻, SeH⁻, CN⁻.

3. The method according to claim 1 or 2, **characterized in that** an amino acid is obtained selected from the group consisting of methionine and selenomethionine.

4. The method according to claim 1 or 2, **characterized in that** homocysteine and the dimer thereof are obtained.

5. The method according to any one of claims 1 to 4, **characterized in that** the N-carboxylation is achieved in a polar aprotic solvent.

6. The method according to claim 5, **characterized in that** the solvent is selected from the group consisting of dimethyl sulfoxide and N- methylpyrrolidone.

7. The method according to any one of claims 1 to 6, **characterized in that** it is prepared isomer L of the amino acid, the isomer D or the mixtures thereof and particularly the racemate, based on the corresponding form of the 2ABL.

8. The method according to any one of claims 1 to 7, **characterized in that** the 2ABL is obtained from homoserine.

9. The method according to claim 8, **characterized in that** the homoserine is the isomer L and is obtained by microbiological fermentation of sugars of natural origin.

10. The method according to any one of claims 1 to 9, **characterized in that** the salts of the amino acid are selected from the group consisting of sodium, lithium, calcium and zinc salts.

11. N-Carboxyl-2-aminobutyrolactone and the salts thereof.
